(19) **Europäisches Patentamt**

**European Patent Office**

**Office européen des brevets**

(11) Publication number: **0 426 884 B1**

(12) **EUROPEAN PATENT SPECIFICATION**

(45) Date of publication of patent specification: **08.09.93**

(51) Int. Cl.5: **F01M 11/10**, F01M 5/00, F16N 29/00

(21) Application number: **89120485.1**

(22) Date of filing: **06.11.89**

(54) **Apparatus for detecting deterioration of lubricating oil.**

(43) Date of publication of application:
**15.05.91 Bulletin 91/20**

(45) Publication of the grant of the patent:
**08.09.93 Bulletin 93/36**

(84) Designated Contracting States:
**DE FR**

(56) References cited:
**DE-A- 3 519 026
GB-A- 721 066
GB-A- 944 639
US-A- 2 012 613
US-A- 3 213 930**

**PATENT ABSTRACTS OF JAPAN vol. 11, no. 14 (M-553)(2461) 14 January 1987,& JP-A-61 190106 (NISSAN) 23 June 1986**

(73) Proprietor: **KYODO OIL TECHNICAL RE-SEARCH CENTER CO., LTD.
10-1, Toranomon 2-chome
Minato-ku Tokyo(JP)**

(72) Inventor: **Hosonuma, Kunihiko c/o KYODO OIL TECHNICAL
RESEARCH CENTER CO. LTD.17-35,
Niizominami 3-chome
Toda City Saitama Pref.(JP)**
Inventor: **Naito, Yasushi c/o KYODO OIL TECHNICAL
RESEARCH CENTER CO. LTD.17-35,
Niizominami 3-chome
Toda City Saitama Pref.(JP)**
Inventor: **Nishino, Tsutomu c/o KYODO OIL TECHNICAL
RESEARCH CENTER CO. LTD.17-35,
Niizominami 3-chome
Toda City Saitama Pref.(JP)**

(74) Representative: **Lehn, Werner, Dipl.-Ing. et al
Hoffmann, Eitle & Partner, Patentanwälte,
Postfach 81 04 20
D-81904 München (DE)**

## Description

The present invention relates to an apparatus for detecting the deterioration of lubricating oil, which allows a deterioration of lubricating oil to be detected with high accuracy.

With a progressive improvement in performance and output characteristics of internal combustion engines, e.g. diesel or gasoline engines, engine oil is used under even more severe conditions. It is thus important to properly determine the timing when the engine oil is to be exchanged and, to this end, necessary to precisely detect progressive deterioration state of the engine oil, in order to prevent massive generation of sludge or steep rise of the viscosity (so-called oil thickening) or the like, which may result in engine malfunctions.

Conventionally, the deterioration of lubricating oil including the engine oil is indicated by physical and/or chemical parameters such as viscosity, total acid value, total basicity and the like. Among others, an especially important and typical parameter is the viscosity, so that a number of methods of quantitatively measuring the viscosity of various lubricating oils are known. A method which is generally carried out makes use of a viscosimeter to measure the viscosity of the lubricating oil sampled from an oil pan. However, this method requires not only use of elaborate viscosimeter and temperature control means, but also a time-consuming sampling operation to be performed, which accompanies a substantial oil loss. Thus, it is difficult to actually apply the method in a monitoring system which is adapted to monitor and indicate the progressive deterioration state of the lubricating oil.

Another apparatus for measuring the viscosity of lubricating oil to detect its deterioration state is disclosed, e.g. in Japanese Patent Application Laid-open Publication No. 59-13,193, wherein an impeller is disposed within an oil passage on the upstream side of the oil pan, with the impeller shaft extending through the wall of the passage and connected with an electric motor arranged outside which, in turn, is connected with an integrating instrument via a reduction gear train. Based on a principle that the revolutional speed of the motor varies depending upon the viscosity of the lubricating oil, the known apparatus is to indicate the deterioration state of the lubricating oil by integrated value of the motor speed.

However, from a practical viewpoint, the above-mentioned known apparatus suffers from various drawbacks as follows. First of all, the apparatus includes various movable components, and is thus relatively complicated and costly in structure and often difficult to achieve high accuracy and satisfactory operational reliability without mechanical failures for a long period. Moreover, since the integrating output signal is a mechanical signal, but not electrical one, it is not always possible to readily dispose the display of the integrating instrument within the visual field of the operator, and the operator may not observe the indication of an excessively deteriorated state of the lubricating oil. Consequently, the known apparatus cannot be suitably applied to a practical monitoring system for indicating the progressive deterioration state of the lubricating oil.

A further known apparatus is shown in DE-A-3 519 026, and includes all the features of the preamble of claim 1. In this instance, however, oil pressure sensors are arranged on upstream and downstream sides of an oil filter, producing a single differential signal dependent on both oil viscosity and the degree of clogging of the filter. Accordingly, neither factor can be distinguished from the other.

Accordingly, it is an object of the present invention to eliminate the above-mentioned drawbacks and provide a novel apparatus which is capable of detecting the deterioration state of lubricating oil with high accuracy and satisfactory operational reliability, and which is simple and less-costly in structure.

To this end, according to the present invention, there is provided an apparatus for detecting the deterioration state of a lubricating oil in a pressure-feed type lubricating oil supply system including a lubricating oil passage with a flow resistance portion, comprising:

means for detecting a differential pressure across the up- and downstream sides of said flow resistance portion; and

signal processing means for generating a signal representing the deterioration state of the lubricating oil in accordance with the viscosity of the lubricating oil;

characterized by means for generating a signal representing the viscosity of the lubricating oil in accordance with the detected differential pressure; and in that said flow resistance portion within the lubricating oil passage comprises cooling pipes of a relatively small diameter formed in an oil cooler;

and in that said signal processing means is operable to evaluate the viscosity of the lubricating oil in accordance with the signal representing the viscosity.

The above-mentioned pressure-feed type lubricating oil system is used in a number of internal combustion engines, such as gasoline engine, diesel engine or the like, in which the lubricating oil stored within the oil pan is supplied to each portion which requires a lubrication using a discharge pressure of a pump. In such a lubricating oil supply system, a small diameter portion within the passage, if any, constitutes a flow resistance causing a differential pressure P across the up- and downstream sides thereof. The differential pressure P is in close relationship in a quantitative sense with the viscosity $\mu$ of the lubricating oil flowing through the passage and, in case of a laminar flow through a tube with a circular cross-section, the relationship is expressed by the following equation (1):

$$Q = (\pi D^4/128\mu) \times P/L \qquad (1)$$

where Q is the flow rate, D the tube diameter and L the tube length.

In the equation (1), since the flow rate Q, tube diameter D and the tube length L can be each considered to be a known variable, the viscosity $\mu$ of the lubricating oil can be evaluated by measuring the differential pressure across the up- and downstream sides of the flow resistance portion in the passage through which the lubricating oil flows during the operation of the engine. On the other hand, as mentioned hereinbefore, there exists a definite relationship between the deterioration and the viscosity of the lubricating oil. Moreover, the viscosity of the lubricating oil tends to increase during a long term use.

Thus, the present invention is based on a recognition that the change in the differential pressure generated across the flow resistance portion of the passage may be used as a parameter precisely indicating the change in the viscosity of the lubricating oil or the progressive deterioration state thereof.

More particularly, according to the present invention, the differential pressure generated across the flow resistance portion in the lubricating oil passage of the pressure-feed type lubricating oil supply system is detected as an electrical signal, which is processed in a signal processing circuit to generate data corresponding to the prevailing viscosity. An output signal representing the deterioration state can be emitted in accordance with the data on the viscosity, and the deterioration state is displayed in accordance with this output signal.

Although various locations within the lubricating oil passage may be used as the flow resistance portion capable of generating the differential pressure, it is particularly advantageous to form the flow resistance portion by an oil cooler disposed in the lubricating oil passage. Such an oil cooler is usually disposed immediately after an oil pump so that a large amount of lubricating oil flows therethrough, and includes cooling pipes of a small diameter adapted to exert to the lubricating oil a substantial flow resistance so that a sufficient differential pressure can be detected across the up- and downstream sides of the cooling pipes. Moreover, the temperature of the lubricating oil can be stabilized by the oil cooler thereby minimizing the fluctuation of the viscosity. Thus, with the apparatus of the present invention which is adapted to detect the differential pressure of a substantial level across the oil cooler, it is possible to achieve the desired detection with an improved accuracy with less susceptibility to external noises (e.g. bubbles in the oil). Furthermore, since the oil cooler is mounted on the outside of an engine block, it is readily possible to attach a differential pressure sensor to the oil cooler as the differential pressure detecting means.

The present invention will now be described in further detail by referring to some preferred embodiments illustrated in the accompanying drawings, in which:

Fig. 1a is a schematic sectional view of an apparatus for monitoring the deterioration of the lubricating oil according to one embodiment of the present invention, in which a differential pressure sensor is secured to an oil cooler;

Fig. 1b is a longitudinal-sectional view of the differential pressure sensor shown in Fig. 1a;

Fig. 1c is a block diagram of a signal processing circuit;

Fig. 2 is a graph illustrating the relationship between the differential pressure and the viscosity of the lubricating oil, obtained by experiments using the apparatus of Figs. 1a to 1c;

Figs. 3a and 3b are respectively schematic views corresponding to Figs. 1a and 1b, illustrating another embodiment of the differential pressure sensor which includes a diaphragm;

Fig. 4 is graph illustrating the relationship between the differential pressure and the viscosity of the lubricating oil, obtained by experiments using the apparatus of Figs. 3a and 3b;

Figs. 5a to 5c are respectively schematic views illustrating various embodiments of the differential pressure sensor;

Fig. 6a is a longitudinal-sectional view showing a known bypass valve in a conventional oil cooler; and

Figs. 6b and 6c are respectively longitudinal-sectional view similar to Fig. 6a, but showing modified embodiments of the differential pressure sensor according to the present invention.

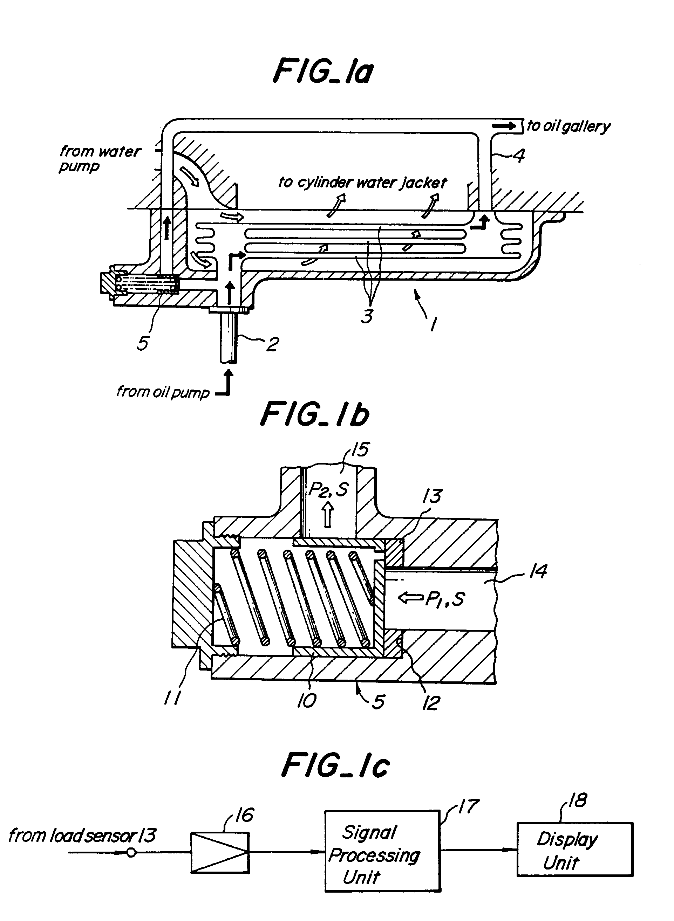

There is shown in Figs. 1a to 1c an apparatus according to a first embodiment of the present invention in which a progressive deterioration state of the lubricating oil is monitored by detecting a differential pressure generated in a lubrication oil passage of an oil cooler which is included in a pressure-feed type oil lubrication system, known *per se*. This type of oil lubrication system is typically arranged such that the lubricating oil, which is stored within an oil pan of an internal combustion engine, is fed under pressure by means of an oil pump, and the total amount of the lubricating oil is supplied to the oil cooler 1. More particularly, the lubricating oil discharged from the oil pump is supplied to the oil cooler 1 through an inlet conduit 2 of a relatively large diameter and flows through cooling pipes 3 of a relatively small diameter where it is cooled down. To this end, water or the like coolant discharged from a coolant pump of a radiator is passed through the oil cooler 1 to flow around the cooling pipes 3 and to thereby cool the cooling pipes 3. Upon being cooled, the lubricating oil is discharged to an oil gallery through an outlet conduit 4 of a large diameter.

The oil cooler 1 further includes a bypass passage which extends between the inlet conduit 2 communicating with the oil pump and the outlet conduit 4 communicating with the oil gallery, in parallel with the cooling pipes 3. A bypass valve 5 is arranged in the bypass passage so that the bypass passage is brought into communication with the inlet conduit 2 via the bypass valve 5 on its upstream side, and with the oil gallery on the downstream side. Since, in order to increase the heat radiation surface area of the cooling pipes 3, the cooling pipes 3 are of a relatively small diameter as compared with the inlet and outlet conduits 2 and 4, the cooling pipes 3 constitute a major flow resistance portion which causes a differential pressure at a substantial level to be generated across the up- and downstream sides of the cooling pipes 3. The differential pressure generated across both ends of the cooling pipes 3 appears, as it is, at the up- and downstream sides of the bypass valve 5.

Fig. 1b shows a detailed arrangement of the bypass valve 5, which is provided with a valve body 10 resiliently urged by a compression spring 11 toward the right side in the figure. An annular load sensor 13 is interposed between the valve body 10 and a valve seat surface 12. The load sensor 13 is adapted to generate an electrical output signal representing the load applied by the valve body 10 to the valve seat surface 12.

Assuming that a pressure within a passage 14 on the upstream side of the bypass valve 5 be $P_1$ and its cross-sectional surface area S, while a pressure within a passage 15 on the downstream side be $P_2$ and its cross-sectional surface area S, and a load of the compression spring to be $W_0$ and a load acting on the load sensor 13 to be W, then the following equation (2) can be obtained.

$$W_0 + SP_2 - SP_1 = W \qquad (2)$$

In this case, if the differential pressure is $\delta P$, then

$$\delta P = P_1 - P_2 = 1/S \cdot (W_0 - W) \qquad (3)$$

In the equation (3), since the cross-sectional surface area S of the passages 14 and 15 and the load $W_0$ of the compression spring 11 can be each considered to be a known variable, the differential pressure $\delta P$ which appears across the bypass valve 5 can be evaluated by measuring the load W acting on the load sensor 13.

Thus, as shown in Fig. 1c, the output signal from the load sensor 13 is supplied to an amplifier 16, and is subsequently supplied to a signal processing unit 17. The data corresponding to the pressure load $W_0$ applied by the compression spring 11 and the cross-sectional surface area S of the passages 14, 15 are previously stored in the signal processing unit 17. Hence, the differential pressure $\delta P$ may be calculated in accordance with the electrical output signal from the load sensor 13. Also, the data corresponding to various parameters in the equation (1) are stored in the signal processing unit 17 to evaluate the viscosity $\mu$ of the lubricating oil from the calculated differential pressure $\delta P$. Moreover, the data representing the relationship between the viscosity $\mu$ and the deterioration, too, are stored in the signal processing unit 17 to generate, based on the calculated value of the viscosity $\mu$, an output signal indicating the deterioration state of the lubricating oil. The relationship between the viscosity $\mu$ and the deterioration state can be obtained e.g. by experimentally evaluating the relationship between the operating time of the engine and the viscosity $\mu$. Incidentally, the viscosity $\mu$ of the lubricating oil varies with the oil temperature, so that data required to compensate for the fluctuation of the oil temperature may also be stored in the signal processing unit 17.

The output signal from the signal processing unit 17 is supplied to a display unit 18 to display the deterioration state of the lubricating oil. The display itself may be made either in digital or analog form, and the viscosity value obtained at the signal processing unit 17 may be displayed as it is. Moreover, when the deterioration proceeds beyond a predetermined limit, a pilot lamp may be continuously lighted, or alternately turned on and off, to visually display an alarm signal.

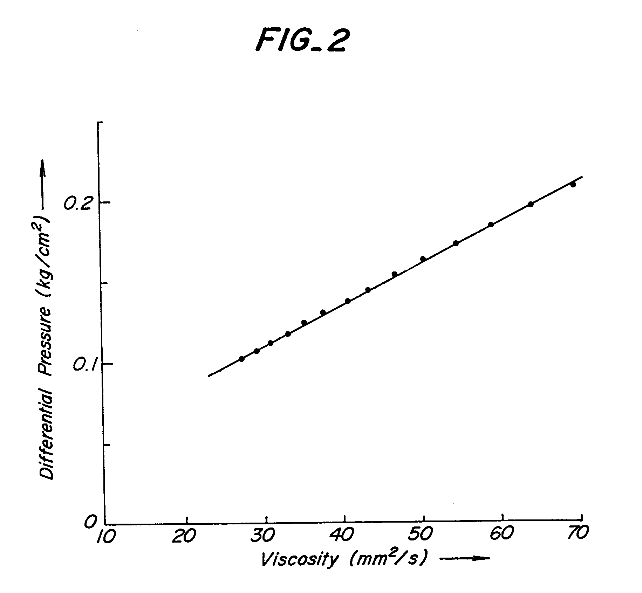

Fig. 2 is a graph illustrating the relationship between the viscosity and the differential pressure measured for the lubricating oils having different viscosity values, obtained by using the above-described sensor. Here, the abscissa and the ordinate respectively scale the viscosity and the differential pressure detected. The viscosity of the lubricating oil has been varied by changing the oil temperature. As shown in Fig. 2, it is apparent that the detected differential pressures are at a significantly high level and exhibit clearly an excellent linear relationship relative to the viscosity, with a high increment of the differential pressure value with respect to the variation in the viscosity. These examinations reveal that, by detecting the differential pressure generated across the oil cooler, it is possible to high accurately detect the viscosity of the lubricating oil.

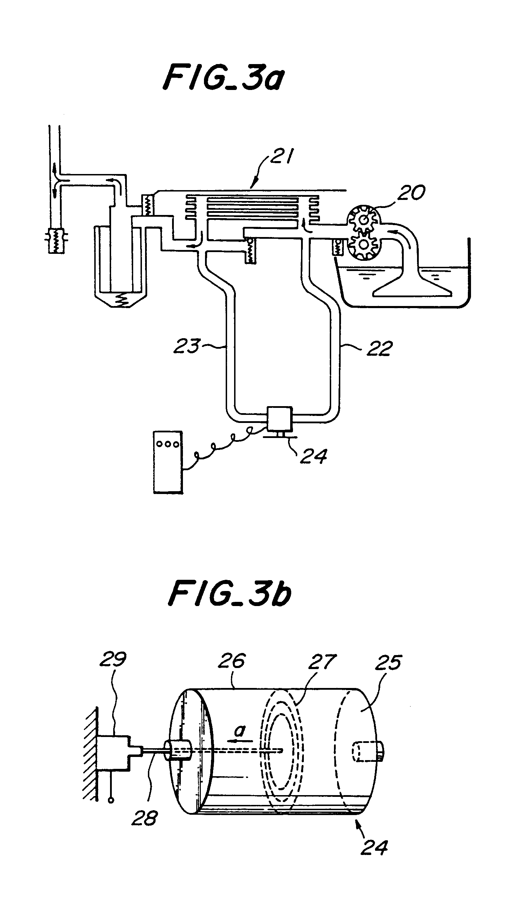

There is shown in Figs. 3a and 3b an apparatus according to another embodiment of the present invention, wherein a lubricating oil pump 20 is connected on its adjacent upstream side to an oil cooler 21, and the differential pressure generated across the oil cooler 21 is detected by means of a diaphragm pressure sensor. The oil cooler 21 is associated with a first bypass passage 22 communicating with a conduit on the upstream side of the oil cooler 21 and a second bypass passage 23 communicating with a conduit on the downstream side thereof, and a diaphragm pressure sensor 24 is interposed between these bypass passages 22, 23. The diaphragm pressure sensor 24 comprises a first pressure chamber 25 communicating with the bypass passage 22 on the upstream side, a second pressure chamber 26 communicating with the bypass passage 23 on the downstream side, and a diaphragm 27 disposed therebetween. When a pressure difference appears across the up- and downstream sides of the diaphragm 27, the central portion of the diaphragm 27 is displaced in the direction of arrow A in Fig. 3b by an amount corresponding to the differential pressure. Consequently, by connecting one end of an operating rod 28 to the central portion of the diaphragm 27 and the other end of the rod 28 is to a differential transformer 29, it is possible to generate by the differential transformer 29 an electrical signal corresponding to the differential pressure generated across the diaphragm 27. The output signal from the differential transformer 29 is processed by means of a signal processing unit to evaluate the viscosity of the lubricating oil.

The result of experiments conducted by the inventor by using the apparatus shown in Figs. 3a and 3b will explained below. A commercially available diesel engine filled with a fresh engine oil in its oil pan has been placed into a long term operation. The engine was operated at a speed of 4,000 rpm and, immediately after starting the operation and after every subsequent 100 hours (i.e., 0, 100, 200 and 300 hours, etc.), the differential pressure and the oil temperature were measured with the engine speed lowered to 800 rpm, while simultaneously sampling the lubricating oil to measure the viscosity using a viscosimeter. The differential pressure was measured using a diaphragm pressure sensor as shown in Figs. 3a and 3b.

The result is shown in the following Table 1.

Table 1

| Operating time | | 0 Hr | 100 Hr | 200 Hr | 300 Hr |
|---|---|---|---|---|---|
| Viscosity [mm$^2$/sec] | 40°C | 109.5 | 139.4 | 176.7 | 195.3 |
| | 100°C | 14.47 | 17.56 | 21.30 | 23.91 |
| Viscosity index | | 135 | 139 | 143 | 151 |
| Oil temperature [°C] | | 89 | 91 | 90 | 91 |
| Engine speed [rpm] | | 802 | 798 | 805 | 796 |
| Viscosity at the measured oil temperature [mm$^2$/sec] | | 19.08 | 22.11 | 27.74 | 30.28 |
| Differential pressure [g/cm$^2$] | | 82 | 89 | 104 | 109 |

In Table 1 above, the viscosity at temperatures of 40°C and 100°C denotes the values as measured according to the measuring method specified in JIS (Japanese Industrial Standard), while the oil temperatures, engine speed, viscosities and the differential pressures at the measured oil temperature are the values obtained by the measurement using the apparatus shown in Figs. 3a and 3b.

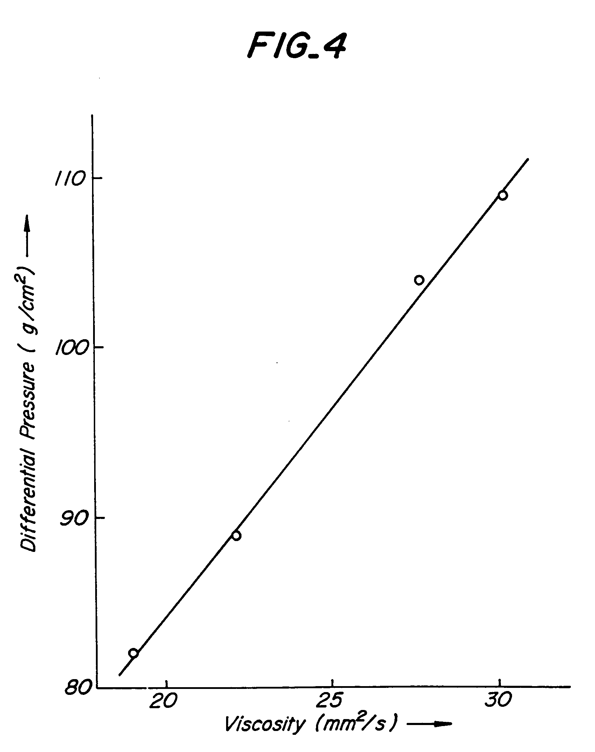

With regard to the viscosity, first of all, it exhibits a linear increase according to the operating time of the engine. The viscosity at the measured oil temperatures in Table 1 denotes values obtained by calculating from the viscosities at 40°C and 100°C, which are usually selected in case of the engine oil, and also from the actually monitored oil temperatures. As to the differential pressure, it should be noted that the measured differential pressure lies within a range of 82 to 109 g/cm$^2$ which, as such, is at a sufficiently high level. Besides, the differential pressure also exhibits a substantially linear increase with respect to the engine operating time. From the relationship between the differential pressure and the viscosity shown in Fig. 4, it is apparent that there exists a linear relationship between the viscosity and the monitored

EP 0 426 884 B1

differential pressure value. In addition, the oil temperature is also approximately constant, and the apparatus does not practically suffer from any adverse effect caused by the fluctuation of the oil temperature. From these results, it can be seen that the detection of the differential pressure allows the viscosity of the lubricating oil to be accurately detected, and a progressive deterioration state of the lubricating oil to be monitored with high accuracy from the detected data on the viscosity. By previously storing in the signal processing unit 17 the data representing the relationship between the differential pressure and the viscosity, which can be obtained experimentally, it is also possible to evaluate the viscosity in accordance with the differential pressure value measured during the monitoring, as well as the above-mentioned stored data as the calibration data.

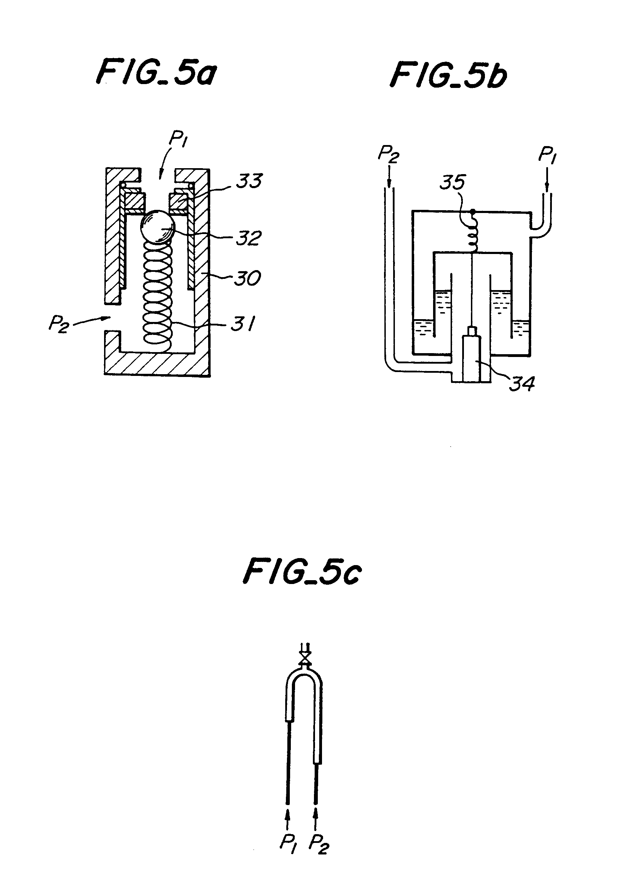

Other embodiments of the differential pressure sensor will be explained below with reference to Figs. 5a to 5c. A load cell is illustrated in Fig. 5a as being integrally incorporated within the bypass valve. Here, the sensor includes a housing 30 accommodating therein a compression spring 31 and a spherical valve body 32, as well as a load cell 33 fixedly secured opposite to the valve body 32. With such an arrangement, since the load acting on the load cell 33 varies depending upon the difference between the pressure $P_1$ on the upstream side and the pressure $P_2$ on the downstream side, it is possible to accurately detect the viscosity of the lubricating oil corresponding to the differential pressure. A bell-mouth type pressure sensor shown in Fig. 5b, in which a differential transformer 34 is supported by a spring 35, or a liquid column type pressure sensor shown in Fig. 5c can also be used.

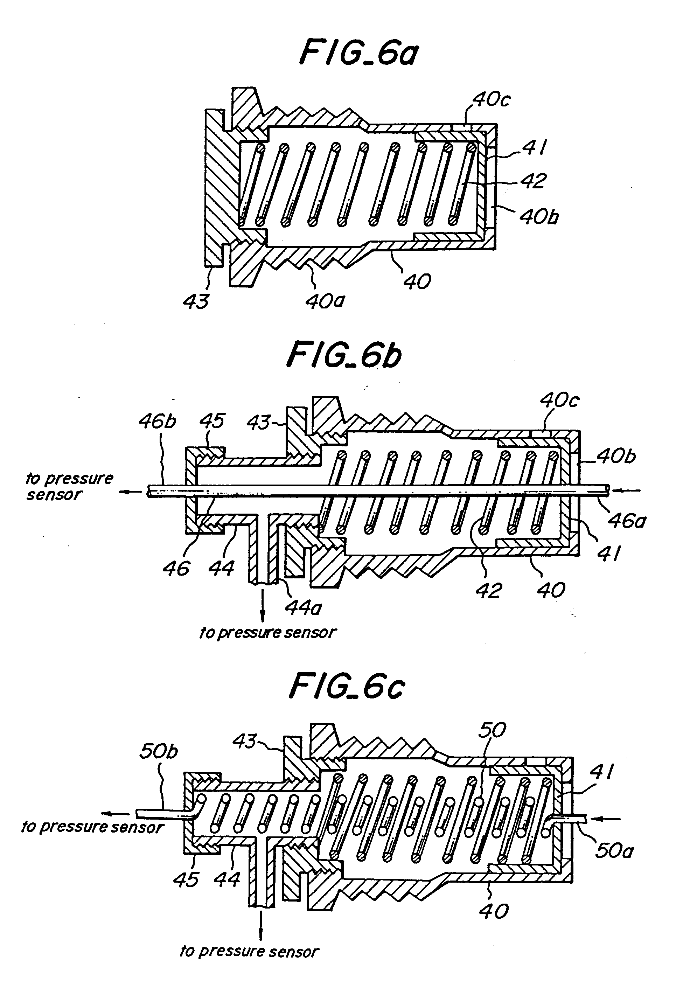

The detection of the differential pressure may be carried out using an existing bypass valve generally included in a commercially available oil cooler. As shown more particularly in Fig. 6a, a bypass valve usually includes a sleeve 40 which is screwed into an oil cooler housing with an external thread 40a formed on its outer peripheral surface. A valve element 41 and a compression spring 42 are accommodated within the internal space of the sleeve 40, with the spring 42 being compressed by an end cap 43. The sleeve 40 has an opening 40b communicating with a passage on the upstream side of the oil cooler, and an opening 40C communicating with a passage on the downstream side of the oil cooler and also with the internal space of the valve element 41. Thus, an existing bypass valve of the above-mentioned structure can be readily modified so as to permit the detection of the difference between the pressure at the opening 40b of the sleeve 40 and that of the internal space of the valve element 41, which corresponds to the differential pressure across the upand downstream sides of the oil cooler.

Another embodiment of the present invention is shown in Fig. 6b, in which the above-mentioned bypass valve has been modified to detect the differential pressure across the up- and downstream sides of the oil cooler. In this embodiment, the end cap 43 has a through hole with an internal thread, and an adapter 44 is screwed into the internal thread of the end cap 43 and brought into communication with the downstream side of the oil cooler via the opening 40c, while a second end cap 45 is screwed into the end portion of the adapter 44. An outlet port 44a is formed in the adapter 44, which is connected to one of the inlet ports of a conventional pressure sensor (not shown). Besides, through holes are each formed axially through the valve element 41 and the second end cap 45 to pass a linear tube 46 made of polytetrafluoroethylene (commercially available under the trademark "TEFLON") therethrough. One end 46a of the tube 46 is brought into communication with a passage on the upstream side of the oil cooler through the opening 40b of the sleeve 40, while the other end 46b of the tube 46 is led to the other inlet port of the pressure sensor. The linear tube 46 may be replaced by a spiral tube 50, as shown in Fig. 6c, which communicates with a passage on the upstream side of the oil cooler on its one end 50a, and with the pressure sensor on another end 50b. Thus, using a commercially available oil cooler and carrying out a simple machining to the end cap 43 of the existing bypass valve, it is readily possible to detect the differential pressure across the up- and downstream sides of the oil cooler.

The present invention is not limited to the above-described embodiments, which have been presented by way of examples only, and various modifications and variations are possible.

For example, although in the above-described embodiments the viscosity of lubricating oil is detected by the differential pressure between the pressures at the up- and downstream sides of the cooling pipes of the oil cooler mounted on a diesel engine, the present invention is not restricted to the lubricating oil supply system for vehicles, and can be applied to all the pressure-feed type lubricating oil supply systems included in various internal combustion engines or other types of machines, having a proper flow resistance portion in the lubricating oil passage.

When the present invention is applied to a pressure-feed type lubricating system without an oil cooler, as in a gasoline engine car, any adverse effect arising from the fluctuation of the oil temperature can be mitigated by conducting the monitoring in a steady operational state of the engine in which the oil temperature is usually kept substantially constant.

EP 0 426 884 B1

It will be readily appreciated from the foregoing description that, according to the present invention, the differential pressure generated across a flow resistance portion of the pressure-feed type lubricating oil supply system can be detected as an electrical signal to evaluate the viscosity of the lubricating oil from the detected differential pressure. The present invention provides an apparatus which is capable of monitoring the deterioration state of the lubricating oil without sampling the oil, which makes it possible to highly accurately detect the viscosity of the lubricating oil, and hence its deterioration state, and which is readily adapted to display the deterioration state of the lubricating oil within a sight of the operator and, by adding an alarm function, to positively and exactly indicate the exchange timing of the lubricating oil.

Reference signs in the claims are inserted for explanatory purpose only.

## Claims

1. An apparatus for detecting the deterioration state of a lubricating oil in a pressure-feed type lubricating oil supply system including a lubricating oil passage (2,4) with a flow resistance portion (3), comprising:
   means (10,13,27,29,32,33,34,41) for detecting a differential pressure across the up- and downstream sides of said flow resistance portion;
   means (17) for generating a signal representing the viscosity of the lubricating oil in accordance with the detected differential pressure; and
   signal processing means (17) for generating a signal representing the deterioration state of the lubricating oil in accordance with the viscosity of the lubricating oil;
   characterized in that said flow resistance portion within the lubricating oil passage comprises cooling pipes (3) of a relatively small diameter formed in an oil cooler (1,21);
   and in that said signal processing means (17) is operable to evaluate the viscosity of the lubricating oil in accordance with the signal representing the viscosity.

2. The apparatus as claimed in Claim 1, characterized in that said means for detecting the differential pressure comprises a bypass valve (5) of said oil cooler (1), and a load sensor (13) for detecting a load acting on the valve seat surface (12) of said bypass valve.

3. The apparatus as claimed in Claim 1, characterized in that said means for detecting the differential pressure comprises a pressure sensor for detecting a difference in pressure between the up- and downstream sides (2,4) of said oil cooler (1).

4. The apparatus as claimed in any one of Claims 1 to 3, characterized by display means (18) for displaying the deterioration state of the lubricating oil based on said signal representing said deterioration state of the lubricating oil.

5. An internal combustion engine comprising apparatus for detecting the deterioration state of a lubricating oil in a pressure-feed type lubricating oil supply system as claimed in any of claims 1 to 4.

## Patentansprüche

1. Gerät zur Erfassung des Verschlechterungszustandes eines Schmieröls in einem druckgespeisten Schmierölversorgungssystem, das einen Schmieröldurchtrittskanal (2, 4) mit einem Strömungswiderstandsabschnitt (3) aufweist, und das aufweist:
   Einrichtungen (10, 13, 27, 29, 32, 33, 34, 41) zur Erfassung des Differenzdruckes zwischen den stromaufwärts- und stromabwärtsgelegenen Seiten des Strömungswiderstandsabschnittes;
   Einrichtungen (17) zur Erzeugung eines Signals, das die Viskosität des Schmieröls entsprechend dem erfaßten Differenzdruck darstellt; und
   Signalverarbeitungseinrichtungen (17) zur Erzeugung eines Signals, das den Verschlechterungszustand des Schmieröls entsprechend der Viskosität des Schmieröls darstellt;
   dadurch **gekennzeichnet,**
   daß der Strömungswiderstandsabschnitts innerhalb des Schmieröldurchtrittskanals Kühlrohre (3) mit einem relativ schmalen Durchmesser umfaßt, die in einem Ölkühler angebracht sind; und
   daß die Signalverarbeitungseinrichtungen (17) für die Berechnung der Viskosität des Schmieröls in Übereinstimmung mit dem die Viskosität darstellenden Signal ausgelegt ist.

7

**2.** Gerät nach Anspruch 1, dadurch gekennzeichnet, daß die Einrichtung zur Erfassung des Differenzdruk-kes ein Bypaßventil (5) des Ölkühlers (1), und einen Belastungssensor (13) zur Erfassung der auf die Ventilsitzoberfläche (12) des Bypaßventils wirkende Belastung aufweist.

**3.** Gerät nach Anspruch 1, dadurch gekennzeichnet, daß die Einrichtung zur Erfassung des Differenzdruk-kes einen Drucksensor zur Erfassung der Druckdifferenz zwischen den stromaufwärts und stromabwärts gelegenen Seiten (2, 4) des Ölkühlers (1) umfaßt.

**4.** Gerät nach einem beliebigen Anspruch 1 bis 3, gekennzeichnet durch eine Anzeigeeinrichtung (18) zum Anzeigen des Verschlechterungszustandes des Schmieröls auf der Basis des den Verschlechterungszustand des Schmieröls darstellenden Signals.

**5.** Verbrennungsmotor mit einem Gerät zur Erfassung des Verschlechterungszustandes eines Schmieröls in einem druckgespeisten Schmierölversorgungssystem nach einem beliebigen Anspruch 1 bis 4.

**Revendications**

**1.** Appareil de détection de l'état de détérioration d'une huile lubrifiante dans un circuit d'alimentation en huile lubrifiante du type à alimentation sous pression, comprenant un passage (2, 4) d'huile lubrifiante présentant une partie (3) qui introduit une résistance à l'écoulement, comprenant :

un dispositif (10, 13, 27, 29, 32, 33, 34, 41) destiné à détecter une pression différentielle entre les côtés amont et aval de la partie introduisant une résistance à l'écoulement,

un dispositif (17) destiné à créer un signal représentant la viscosité de l'huile lubrifiante d'après la pression différentielle mesurée, et

un dispositif (17) de traitement de signaux destiné à créer un signal qui représente l'état de détérioration de l'huile lubrifiante en fonction de la viscosité de celle-ci,

caractérisé en ce que la partie introduisant une résistance à l'écoulement dans le passage d'huile lubrifiante comprend des tubes de refroidissement (3) de diamètre relativement petit, formés dans un refroidisseur (1, 21) d'huile, et

en ce que le dispositif (17) de traitement de signaux est destiné à évaluer la viscosité de l'huile lubrifiante d'après le signal représentant la viscosité.

**2.** Appareil selon la revendication 1, caractérisé en ce que le dispositif destiné à détecter la pression différentielle comporte une soupape de dérivation (5) du refroidisseur (1) d'huile, et un capteur (13) de charge destiné à détecter une charge qui agit sur la surface (12) de siège d'obturateur de la soupape de dérivation.

**3.** Appareil selon la revendication 1, caractérisé en ce que le dispositif destiné à détecter la pression différentielle comporte un capteur de pression destiné à détecter une différence de pression entre les côtés amont et aval (2, 4) du refroidisseur (1) d'huile.

**4.** Appareil selon l'une quelconque des revendications 1 à 3, caractérisé par un dispositif d'affichage (18) destiné à afficher l'état de détérioration de l'huile lubrifiante d'après le signal représentant l'état de détérioration de l'huile lubrifiante.

**5.** Moteur à combustion interne comprenant un appareil de détection de l'état de détérioration d'une huile lubrifiante, dans un circuit d'alimentation en huile lubrifiante du type à alimentation sous pression, selon l'une quelconque des revendications 1 à 4.

## FIG_Ia

from water pump

to oil gallery

to cylinder water jacket

4

5

2

3

1

from oil pump

## FIG_Ib

15

$P_2, S$

13

14

$\Leftarrow P_1, S$

11

10

5

12

## FIG_Ic

from load sensor 13

16

| Signal Processing Unit | 17

| Display Unit | 18

FIG_2

# FIG_3a

# FIG_3b

# FIG.4

# FIG_5a

$P_1$

33

32

30

31

$P_2$

# FIG_5b

$P_2$

35

$P_1$

34

# FIG_5c

$P_1$ $P_2$

## FIG_6a

## FIG_6b

to pressure sensor

to pressure sensor

## FIG_6c

to pressure sensor

to pressure sensor